# EUROPÄISCHE PATENTSCHRIFT

(11) **EP 0 384 315 B1**
(45) Veröffentlichungstag und Bekanntmachung des Hinweises auf die Patenterteilung: **16.06.1993**
(21) Anmeldenummer: 90103021.3
(22) Anmeldetag: 16.02.1990
(51) Int. Cl.: C25B 3/02

(54) **Verfahren zur Herstellung von Lactonen**
Process for manufacturing lactones
Procédé de production de lactones

(30) Priorität: 21.02.1989 DE 3905243
(43) Veröffentlichungstag der Anmeldung: 29.08.1990
(73) Patentinhaber: BASF Aktiengesellschaft, 67063 Ludwigshafen (DE)
(72) Erfinder: Steiniger, Michael, Dr., D-6730 Neustadt (DE); Hannebaum, Heinz, D-6700 Ludwigshafen (DE)

(56) Entgegenhaltungen:
- FR-A- 2 595 358
- ELECTROCHIMICA ACTA, Band 31, Nr. 1, Januar 1986, Seiten 83,84, Oxford, GB; M. LOPEZ-SEGURA et al.: "Anodic oxidation of 2- and 3-methylcyclohexanones"

## Beschreibung

Diese Erfindung betrifft ein neues Verfahren für die Herstellung von Lactonen durch elektrochemische Oxidation von Lactolen.

Es sind schon verschiedene Verfahren für die Herstellung von Lactonen aus Lactolen beschrieben worden. So ist z.B. aus Helv. Chim. Acta 47, 398-399 (1964) bekannt, daß sich Bromcarbamid für eine derartige Oxidation verwenden läßt. Andere für diesen Zweck eingesetzte Oxidationsmittel sind z.B. Chromverbindungen, wie Chromtrioxid in Pyridin (THL 1975, 2275-2277), Silberoxid (Helv. Chim. Acta 55, 249-252 (1972) oder Silbercarbonat auf Kieselgur (J. Org. Chem. 40, 892-894 (1975).

Diese Methoden sind für eine industrielle Nutzung wenig vorteilhaft. So erfordert der Umgang mit Brom oder Chromsalzen aus Sicherheits- und Umweltgründen einen erheblichen Aufwand und die Verwendung der teuren Silbers als Oxidationsmittel eine zur Vermeidung von Silberverlusten aufwendige Regenerierung.

Es wurde nun ein neues Verfahren gefunden, das es ermöglicht, Lactole nach einem technisch einfachen und besonders umweltfreundlichen Verfahren zu den entsprechenden Lacfonen zu oxidieren.

Nach dem neuen Verfahren dieser Erfindung stellt man Lactone der allgemeinen Formel 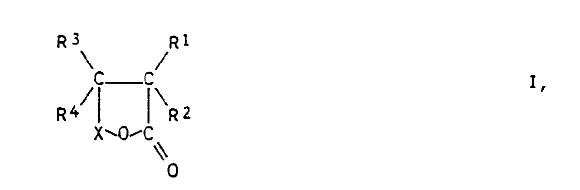
in der X das Brückenglied 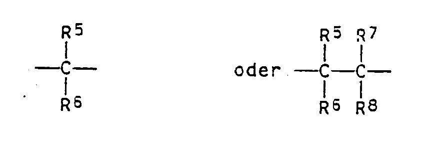
und
R¹ bis R⁸ Wasserstoffatome, Hydroxigruppen, Alkyl- oder Alkylengruppen, die durch Halogenatome, Hydroxigruppen, Carboxigruppen oder Nitrilgruppen substituiert sein können, Alkoxigruppen oder Acetoxigruppen bedeuten, wobei R¹, R³, R⁵ und R⁷ auch für ringförmige Kohlenwasserstoffreste und die beiden an einem C-Atom gebundenen R-Reste auch gemeinsam für einen Alkylenrest oder für ein Sauersfoffatom stehen können, dadurch her, daß man Lactole der allgemeinen Formel 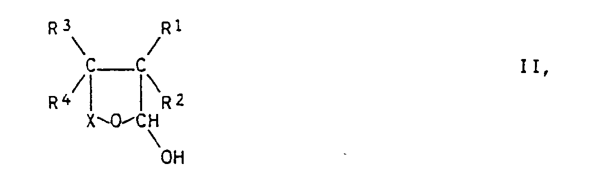
in der X und die Reste R die obengenannte Bedeutung haben, in Gegenwart eines ionogenen Halogenids elektrochemisch oxidiert.

Die als Ausgangsstoffe verwendeten Lactole der Formel II enthalten als Reste R¹ bis R⁸ Wasserstoffatome, Hydroxigruppen, Alkoxigruppen, wie Methoxi- oder Ethoxigruppen, Acetoxigruppen, Alkyl- oder Alkylengruppen, die z.B. 1 bis 12, vorzugsweise 1 bis 8, insbesondere 1 bis 4 C-Atome enthalten, wie Methyl-, Ethyl-, n- und iso-Propyl-, n-Butyl, iso-Butyl-, tert.-Butyl, Vinyl-, Allyl- oder Isopropenylgruppen. Die Alkyl- oder Alkylengruppen können ihrerseits durch Halogenatome wie Fluor- Chlor- oder Bromatome, Hydroxigruppen, Carboxigruppen oder Nitrilgruppen substituiert sein. Solche substituierten Reste sind z.B. Chlormethyl- oder Hydroximethylgruppen. Die Reste R¹, R³, R⁵ und R⁷ können auch ringförmige Kohlenwasserstoffreste, wie Cycloalkylreste, die z.B. 3 bis 8, vorzugsweise 5 bis 6 C-Atome enthalten, wie Cyclopentyl- oder Cyclohexylreste sein. Außerdem können jeweils die beiden paarförmig an einem C-Atom gebundenen R-Reste auch gemeinsam für einen Alkylenrest, wie für einen Rest der Formel -(CH₂)ₙ-, in der n eine ganze Zahl von 4 bis 7 bedeutet, oder für ein Sauersfoffatom stehen. Bevorzugt sind solche Ausgangssfoffe der Formel II, in denen die Reste R Wasserstoffatome, Hydroxigruppen oder Alkyl- oder Alkoxigruppen mit 1 bis 4 C-Atomen oder zwei an einem C-Atom gebundene R-Reste ein Sauersfoffatom bedeuten. Beispielsweise seien die folgenden Verbindungen der Formel II genannt: 2-Hydroxi-4-methyl-tetrahydropyran, 2,5-Dihydroxitetrahydropyran, 3,3-Dimethyl-2-hydroxi-4-oxo-tetrahydrofuran, 2-Hydroxi-5-hydroximethyltetrahydrofuran oder 2,4-Dihydroxi-3-methyltetrahydrofuran.

Die Ausgangsverbindungen der Formel II lassen sich auf technisch einfache und an sich bekannte Weise, z.B. durch Hydroformylierung ungesättigter Alkohole an Rhodium-Kafalysatoren, wie z.B in THL 25, 4051-4053 (1984) beschrieben, herstellen.

Als ionogene Halogenide kommen Salze der Jodwasserstoff-, Bromwasserstoff- und Chlorwasserstoffsäure in Befracht. Bevorzugt sind Salze der Bromwasserstoffsäure, wie Alkali- oder Erdalkalibromide sowie quartäre Ammoniumbromide, insbesondere Tetraalkylammoniumbromide. Das Kation spielt keine erfindungswesentliche Rolle, es können daher auch andere ionogene Metallhalogenide verwendet werden, vorteilhaft wird man jedoch billige Halogenide wählen. Beispielsweise seien Natrium-, Kalium-, Calcium-und Ammoniumbromid sowie Di-, Tri- und Tetramethyl- oder Tetraethylammoniumbromid genannt.

Für die Elektrooxidation verwendet man zweckmäßigerweise eine Lösung der Lactole und des ionogenen Halogenids in einem geeigneten Lösungsmittel. Als Lösungsmittel eignen sich solche, die unter den Elektolysebedingungen ausreichend stabil sind. Das sind z.B. Ether, wie Dioxan, Chlorkohlenwasserstoffe, wie Methylenchlorid, Ketone, wie Aceton, Nitrile, wie Acetonitril oder Alkohole, wie Alkanole mit 1 bis 4 C-Atomen. Besonders gut geeignet sind Methanol und Ethanol.

Die Zusammensetzung des Elektrolyten kann in weiten Grenzen gewählt werden. So besteht der Elektrolyt beispielsweise aus einer alkoholischen Lösung des Lactols der allgemeinen Formel II mit einem Gehalt des Lactols von 1 bis 50, vorzugsweise 3 bis 20 Gew.% und einem Gehalt an dem ionogenen Halogenid von 0,1 bis 20, vorzugsweise 0,5 bis 5 Gew.%.

Das erfindungsgemäße Verfahren kann in den üblichen technischen Elektrolysezellen durchgeführt werden. Man verwendet vorzugsweise ungeteilte Durchflußzellen, die zweckmäßigerweise zur Minimierung der Zellspannung geringe Elektrodenabstände von beispielsweise 0,25-2 mm aufweisen. Als Anoden werden z.B. Edelmetalle wie Platin, mit Edelmetalloxiden dotiertes Titan, Metalloxide, wie MnO₂ und PbO₂, bevorzugt jedoch Graphit eingesetzt. Als Kathodenmaterialien werden z.B. Blei, Eisen, Stahl, Nickel oder Edelmetalle wie Platin verwendet. Bevorzugtes Kathodenmaterial ist ebenfalls Graphit.

Die Stromausbeuten sind bei den erfindungsgemäßen Verfahren ungewöhnlich hoch. So sind die Lactole bei Elektrolyse mit 2 bis 2,5 F/mol Lactol bereits vollständig umgesetzt. Die Stromdichte ist kein begrenzender Faktor, sie beträgt z.B. 0,1 bis 25 A/dm², vorzugsweise 3 bis 15 A/dm².

Man führt das erfindungsgemäße Verfahren z.B. bei Temperaturen bis zu 5°C unter dem Siedepunkt des eingesetzten Lösungsmittels, vorzugsweise bei 5 bis 90°C, insbesondere bei 10 bis 70°C durch. Verwendet man als Lösungsmittel Methanol oder Ethanol, so elektrolysiert man zweckmäßigerweise bei Temperaturen von 15 bis 35°C.

Die Aufarbeitung der Elektrolyseausträge kann man nach an sich bekannten Methoden vornehmen. Zweckmäßigerweise wird der Elektrolyseaustrag destillativ aufgearbeitet. Überschüssiges Lösungsmittel wird zunächst abdestilliert. Die Halogenide kann man z.B. durch Filtration oder Extraktion abtrennen und das Lacton durch Destillation oder Umkristallisation rein gewinnen. Lösungsmittel, evtl. unumgesetztes Lactol sowie Halogenide können vorteilhaft zur Elektrolyse zurückgeführt werden.

Das erfindungsgemäße Verfahren kann sowohl diskontinuierlich als auch kontinuierlich durchgeführf werden.

Die nach dem erfindungsgemäßen Verfahren hergestellten Lactone der allgemeinen Formel I sind vielfältig einsetzbare Zwischenprodukte für die Synthese von Riechstoffen, Aromastoffen und Pharmazeutika.

### Beispiele

Die Elektrooxidationen wurden in einer ungeteilten Elektrolysezelle mit 6 Graphitelektroden (5 Spalte, Elektrodenabstand 0,5 mm) bei Temperaturen von 20 bis 25°C durchgeführt. Während der Elektrolyse wurde der Elektrolyt, der als Leitsalz Natriumbromid enthielt, mit 200 l/h über einen Wärmetauscher durch die Zelle gepumpt.

### Beispiel 1

Elektrosynthese von 2,4-Dioxo-3,3-dimethyltetrahydrofuran

Elektrolyt: 130 g 3,3-Dimethyl-2-hydroxi-4-oxotetrahydrofuran (1) 26 g Natriumbromid und 2 440 g Methanol
Elektrolyse mit 2,5 F/mol 1
Stromdichte: 7,5 A/dm²
Temperatur : 20 bis 21°C

Nach Beendigung der Elektrolyse wird vom Elektrolyseaustrag Methanol bei Normaldruck abdestilliert, das ausgefallene Salz bei Raumtemperatur über eine Drucknutsche abgetrennt (kann zusammen mit Methanol zur Elektrolyse zurückgeführt werden) und das Filtrat bei 60 bis 70°C und 5 bis 6 mbar reindestilliert. Man erhält 88 g 2,4-Dioxo-3,3-dimethyltetrahydrofuran entsprechend einer Materialausbeute von 69 %.

### Beispiel 2

### Elektrosynthese von 2-Oxo-4-methyltetrahydropyran

Elektrolyt: 250 g 2-Hydroxi-4-methyltetrahydrofuran (2), 25 g Natriumbromid und 2 225 g Methanol
Elektrolyse mit 2,5 F/mol 2
Stromdichte: 10 A/dm²
Temperatur : 21°C

Die Isolierung des Produktes nach der Elektrolyse erfolgt analog Beispiel 1. Man erhält nach einer Reindestillation bei 119 bis 120°C und 20 mbar 168,8 g 2-Oxo-4-methyltetrahydropyran entsprechend einer Ausbeute von 68 %

### Beispiel 3

### Elektrosynthese von 5-Hydroximethyl-2-oxotetrahydrofuran

Elektrolyt: 212 g eines 1:1-Gemisches aus 2-Hydroxi-5-hydroximethyltetrahydrofuran (3a) und 2,5-Dihydroxitetrahydropyran (3b), 26 g Natriumbromid und 2 209 g Methanol.
Elektrolyse mit 2,0 F/mol 3
Stromdichte 10 A/dm²
Temperatur 21 bis 23°C

Der Austrag wird analog Beispiel 1 aufgearbeitet und das Rohprodukt bei 100-103°C und 0,1 mbar reindestilliert. Man isoliert 175,4 g 5-Hydroximethyl-2-oxotetrahydrofuran entsprechend einer Ausbeute von 84 %.

## Patentansprüche

1. Verfahren zur Herstellung von Lactonen der allgemeinen Formel 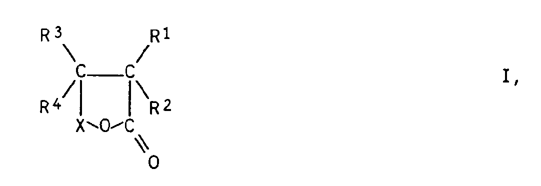 in der X das Brückenglied 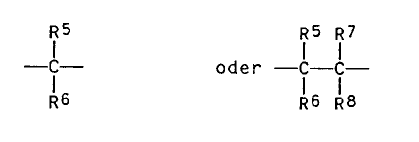 und
R¹ bis R⁸ Wasserstoffatome, Hydroxigruppen, Alkyl- oder Alkylengruppen, die durch Halogenatome, Hydroxigruppen, Carboxigruppen oder Nitrilgruppen substituiert sein können, Alkoxigruppen oder Acetoxigruppen bedeuten, wobei R¹, R³, R⁵ und R⁷ auch für ringförmige Kohlenwasserstoffreste und die beiden an einem C-Atom gebundenen R-Reste auch gemeinsam für einen Alkylenrest oder für ein Sauerstoffatom stehen können, dadurch gekennzeichnet, daß man Lactole der allgemeinen Formel 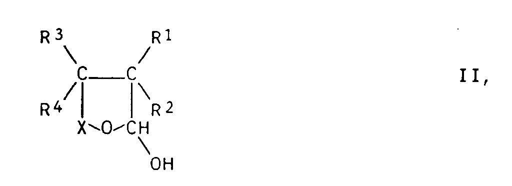 in der X und die Reste R die obengenannte Bedeutung haben, in Gegenwart eines ionogenen Halogenids elektrochemisch oxidiert.

2. Verfahren nach Anspruch 1, dadurch gekennzeichnet, daß man als ionogenes Halogenid ein Salz der Chlor- oder Bromwasserstoffsäure verwendet.

3. Verfahren nach Anspruch 1, dadurch gekennzeichnet, daß man für die elektrochemische Oxidation Graphitanoden verwendet.

4. Verfahren nach Anspruch 1, dadurch gekennzeichnet, daß man für die Elektrooxidation eine Lösung des Lactols der allgemeinen Formel II und des ionogenen Halogenids in einem unter den Elektrolysebedingungen stabilen Lösungsmittel verwendet.

5. Verfahren nach Anspruch 4, dadurch gekennzeichnet, daß man eine alkoholische Lösung des Lactols der allgemeinen Formel II verwendet, die einen Lactolgehalt von 1 bis 50 Gew.-% und einen Halogenidgehalt von 0,1 bis 20 Gew.-% aufweist.

6. Verfahren nach den Ansprüchen 4 und 5, dadurch gekennzeichnet, daß man als Lösungsmittel Methanol oder Ethanol verwendet.

## Claims

1. A process for preparing lactones of the formula 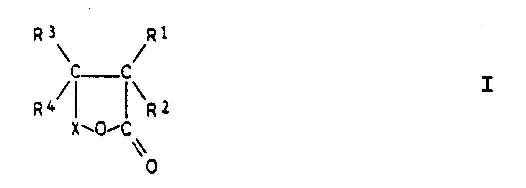 where X is the linker 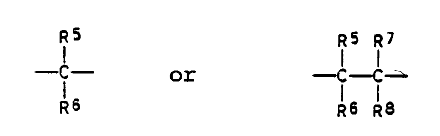 and
R¹ to R⁸ are each hydrogen, hydroxyl, alkyl or alkenyl, which can be substituted by halogen atoms, hydroxyl groups, carboxyl groups or nitrile groups, or are each alkoxy or acetoxy, where R¹, R³, R⁵ and R⁷ can also be cyclic hydrocarbon radicals and the two R radicals bonded to a carbon can also together be an alkylene radical or an oxygen atom, which comprises electrochemical oxidation of lactols of the formula 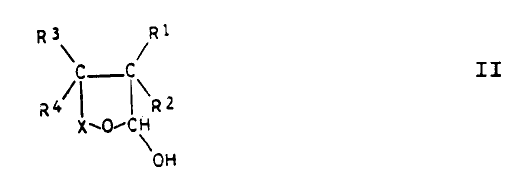 where X and the radicals R have the abovementioned meanings, in the presence of anionic halide.

2. A process as claimed in claim 1, wherein a salt of hydrochloric or hydrobromic acid is used as ionic halide.

3. A process as claimed in claim 1, wherein graphite anodes are used for the electrochemical oxidation.

4. A process as claimed in claim 1, wherein a solution of the lactol of the formula II and of the ionic halide in a solvent which is stable under the electrolysis conditions is used for the electrochemical oxidation.

5. A process as claimed in claim 4, wherein an alcoholic solution of the lactol of the formula II, which has an lactol content of from 1 to 50% by weight and a halide content of from 0.1 to 20% by weight, is used.

6. A process as claimed in claims 4 and 5, wherein methanol or ethanol is used as solvent.

## Revendications

1. Procédé de préparation de lactones de formule générale 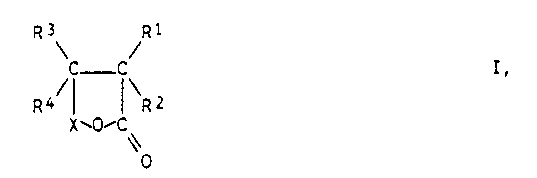 dans laquelle X représente le maillon de pontage 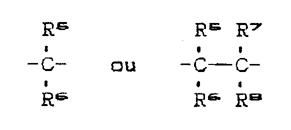 et
R¹ à R⁸ représentent des atomes d'hydrogène, des groupements hydroxy, des groupements alkyle ou alkylène qui peuvent être substitués par des atomes d'halogène, par des groupements hydroxy, par des groupements carboxy ou par des groupements nitrile, des groupements alcoxy ou des groupements acétoxy, R¹, R³, R⁵ et R⁷ pouvant aussi être mis pour des restes hydrocarbonés cycliques et les deux restes R fixés à un atome de carbone pouvant également être mis ensemble pour un reste alkylène ou pour un atome d'oxygène, caractérisé en ce qu'on oxyde par voie électrochimique de: lactols de formule générale 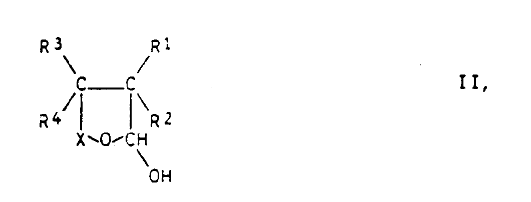 dans laquelle X et les restes R ont les significations sus-indiquées, en présence d'un halogénure ionogène.

2. Procédé selon la revendication 1, caractérisé en ce qu'on utilise, comme halogénure ionogène, un sel de l'acide chlorhydrique ou bromhydrique.

3. Procédé selon la revendication 1, caractérisé en ce qu'on utilise des anodes en graphite pour l'oxydation électrochimique.

4. Procédé selon la revendication 1, caractérisé en ce qu'on utilise, pour l'électro-oxydation, une solution du lactol de formule générale II et de l'halogénure ionogène dans un solvant stable dans les conditions de l'électrolyse.

5. Procédé selon la revendication 4, caractérisé en ce qu'on utilise une solution alcoolique du lactol de formule générale II qui présente une teneur en lactol de 1 à 50% en poids et une teneur en halogénure de 0,1 à 20% en poids.

6. Procédé selon la revendication 4 ou 5, caractérisé en ce qu'on utilise, comme solvant, du méthanol ou de l'éthanol.
